(19) Europäisches Patentamt
European Patent Office
Office européen des brevets

(11) Veröffentlichungsnummer : **0 144 044**
**B1**

(12) # EUROPÄISCHE PATENTSCHRIFT

(45) Veröffentlichungstag der Patentschrift : **02.03.88**

(21) Anmeldenummer : **84114105.4**

(22) Anmeldetag : **22.11.84**

(51) Int. Cl.⁴ : **C 07 C 49/235**, C 07 D249/08, C 07 C 49/563, C 07 C 49/255, C 07 C 49/227, C 07 C 45/45, C 07 C 45/46

(54) **Substituierte Acetylenketone.**

(30) Priorität : **01.12.83 DE 3343531**

(43) Veröffentlichungstag der Anmeldung : **12.06.85 Patentblatt 85/24**

(45) Bekanntmachung des Hinweises auf die Patenterteilung : **02.03.88 Patentblatt 88/09**

(84) Benannte Vertragsstaaten : **BE CH DE FR GB IT LI NL**

(56) Entgegenhaltungen :
**US-A- 3 409 424**
**AGR. BIOL. CHEM., Band 39, Nr. 2, Februar 1975, Seiten 519-527; T. FUKUMARU et al.: "Synthesis and bioactivity of novel acetylenic compounds"**
**HELVETICA CHIMICA ACTA, Band 62, Fasc. 62, Nr. 89, 1979, Seiten 852-865; M. KARPF et al.: "Thermische Cyclisierung von alpha-Alkinonen zu 2-Cyclopenonen"**

(73) Patentinhaber : **BAYER AG**
**Konzernverwaltung RP Patentabteilung**
**D-5090 Leverkusen 1 Bayerwerk (DE)**

(72) Erfinder : **Elbe, Hans Ludwig, Dr.**
**Dasnöckel 59**
**D-5600 Wuppertal 11 (DE)**

**EP 0 144 044 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue substituierte Acetylenketone, ein Verfahren zu deren Herstellung und ihre Verwendung als Zwischenprodukte zur Synthese von Stoffen mit pflanzenwachstumsregulierender und fungizider Wirksamkeit.

Es ist bereits bekannt geworden, daß sich Azolyl-methyl-ketone als Zwischenprodukte zur Herstellung von Azolyl-Derivaten mit pflanzenwuchsregulierenden und fungiziden Eigenschaften verwenden lassen (vgl. EP-B 0 032 200 und EP-B 0 031 911). So kann z. B. durch Umsetzung von 1-(1,2,4-Triazol-1-yl)-3,3-bis-fluormethyl-butan-2-on mit Cyclohexyl-methyl-bromid das 1-Cyclohexyl-2-(1,2,4-triazol-1-yl)-4,4-bis-fluormethyl-pentan-3-on nach folgendem Schema synthetisiert werden :

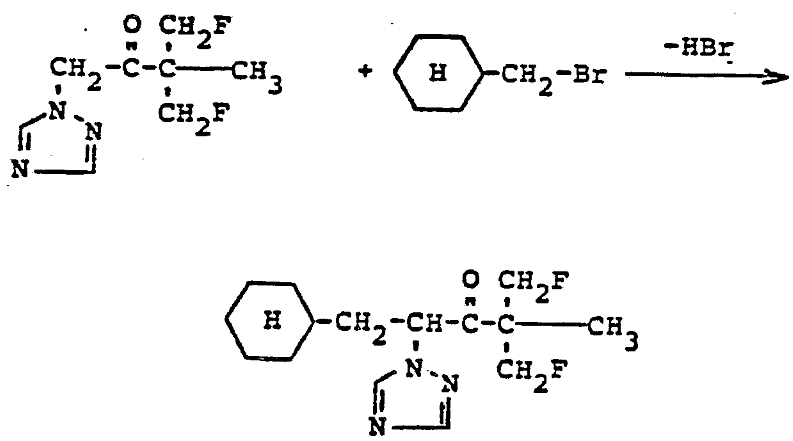


Nachteilig ist jedoch, daß bei dieser Art der Herstellung von Azolyl-Derivaten mit pflanzenwachstumsregulierender und fungizider Wirksamkeit die als Zwischenprodukte benötigten Azolyl-methyl-ketone nur durch mehrstufige Synthesen erhältlich sind und die dabei als Ausgangsstoffe eingesetzten Materialien zum Teil nur schwierig zugänglich sind.

Aus den Artikeln in Agr. Biol. Chem. 39, 519-527 (1975) und Helv. Chim. Acta 62, 852-865 (1979) sind zahlreiche substituierte Acetylenketone bekannt. Es werden jedoch keine Stoffe dieses Typs offenbart, in denen die Keto-Gruppe einen verzweigten, gegebenenfalls substituierten Alkylrest trägt und gleichzeitig die Acetylen-Gruppe mit Alkyl, gegebenenfalls substituiertem Cycloalkenyl, gegebenenfalls substituiertem Cycloalkylalkyl, gegebenenfalls substituiertem Aryl oder gegebenenfalls substituiertem Aralkyl verbunden ist.

Es wurden nun neue substituierte Acetylenketone der Formel (I)

$$R^2—C≡C—CO—R^1 \qquad (I)$$

in welcher

$R^1$ für die Gruppierungen

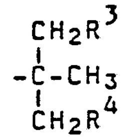 , 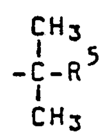 und 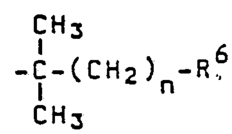 steht,

$R^2$ für geradkettiges oder verzweigtes Alkyl mit 1 bis 6 Kohlenstoffatomen, für jeweils gegebenenfalls einfach oder zweifach, gleich oder verschieden durch Methyl, Ethyl, Isopropyl und/oder tert.-Butyl substituiertes Cyclopropylmethyl, Cyclopentylmethyl, Cyclohexylmethyl, Cyclopentenyl, Cyclohexenyl oder Cycloheptenyl sowie für jeweils gegebenenfalls einfach oder zweifach gleich oder verschieden substituiertes Phenyl oder Phenylalkyl mit 1 bis 2 Kohlenstoffatomen im Alkylteil steht, wobei als Phenylsubstituenten jeweils Fluor, Chlor, Methyl, Ethyl, Isopropyl, tert.-Butyl, Methoxy, Methylthio, Trifluormethyl, Trifluormethoxy, Trifluormethylthio, Nitro, Cyano, Methoxycarbonyl, sowie jeweils gegebenenfalls durch Chlor oder Fluor substituiertes Phenyl und Phenoxy in Frage kommen,

$R^3$ für Fluor oder Chlor steht ;

$R^4$ für Wasserstoff, Fluor oder Chlor steht ;

$R^5$ für geradkettiges oder verzweigtes Alkyl mit 2 bis 6 Kohlenstoffatomen, Vinyl, Propargyl, die —CH=O-Gruppe, Methoximinomethyl, Dimethoxymethyl, den Dioxolan- oder 1,3-Dioxan-Rest steht ;

$R^6$ für Cyano oder gegebenenfalls einfach oder zweifach, gleich oder verschieden substituiertes Phenyl steht, wobei als Substituenten die bei $R^2$ bereits genannten Phenylsubstituenten in Frage

kommen, sowie für die Gruppierungen $—XR^7$ und $—CONR^8R^9$ steht ;

$R^7$ für geradkettiges oder verzweigtes Alkyl mit 1 bis 4 Kohlenstoffatomen, Halogenalkyl mit 1 bis 2 Kohlenstoffatomen und 1 bis 5 gleichen oder verschiedenen Halogenatomen, wie Fluor- und Chloratomen, sowie für jeweils gegebenenfalls einfach oder mehrfach, gleich oder verschieden substituiertes Phenyl und Benzyl steht, wobei jeweils als Substituenten die bei $R^2$ bereits genannten Phenylsubstituenten in Frage kommen ;

$R^8$ für Wasserstoff, Methyl, Ethyl, Isopropyl oder für gegebenenfalls einfach oder zweifach, gleich oder verschieden substituiertes Phenyl steht, wobei als Substituenten die bei $R^2$ bereits genannten Phenylsubstituenten in Frage kommen ;

$R^9$ für Wasserstoff, Methyl oder Isopropyl steht ;

X für O, S, SO oder $SO_2$ steht und der Index

n für die Zahlen 0 oder 1 steht,

gefunden.

Weiterhin wurde gefunden, daß man die neuen substituierten Acetylenketone der Formel (I) erhält, wenn man Acetylen-Derivate der Formel (II)

$$R^2—C{\equiv}C—M \qquad (II)$$

in welcher

$R^2$ die oben angegebenen Bedeutungen hat und

M für ein Metall oder Wasserstoff steht ;

mit Säurederivaten der Formel (III)

$$Y—CO—R^1 \qquad (III)$$

in welcher

$R^1$ die oben angegebenen Bedeutungen hat und

Y für eine elektronenziehende Abgangsgruppierung steht,

gegebenenfalls in Gegenwart eines Verdünnungsmittels, bzw. zusätzlich gegebenenfalls in Gegenwart einer Base und von Cu-(I)-Ionen als Katalysator umsetzt.

Schließlich wurde gefunden, daß die neuen substituierten Acetylenketone der Formel (I) sehr gut geeignet sind als Zwischenprodukte zur Herstellung von Azolyl-Derivaten mit pflanzenwuchsregulierender und fungizider Wirksamkeit.

Überraschenderweise lassen sich pflanzenwuchsregulierend und fungizid wirksame Azolyl-ketone und -carbinole ausgehend von den erfindungsgemäßen substituierten Acetylenketonen der Formel (I) einfacher und in höherer Ausbeute herstellen als nach den bisher bekannten Verfahren, bei dem die entsprechenden Azolylmethyl-ketone als Zwischenprodukte eingesetzt wurden.

Die neuen substituierten Acetylenketone sind durch die Formel (I) allgemein definiert.

Bevorzugt sind Verbindungen der Formel (I), in denen $R^1$ für die Gruppierung $—C(CH_2R^3)(CH_2R^4)CH_3$ steht, wobei $R^3$ für Fluor oder Chlor steht, und $R^4$ für Wasserstoff, Fluor oder Chlor steht.

Verwendet man beispielsweise α-Fluormethyl-α-methylpropionsäurechlorid und das Lithiumsalz des Phenylacetylens als Ausgangsstoffe, so kann der Reaktionsablauf des erfindungsgemäßen Verfahrens durch das folgende Formelschema wiedergegeben werden :

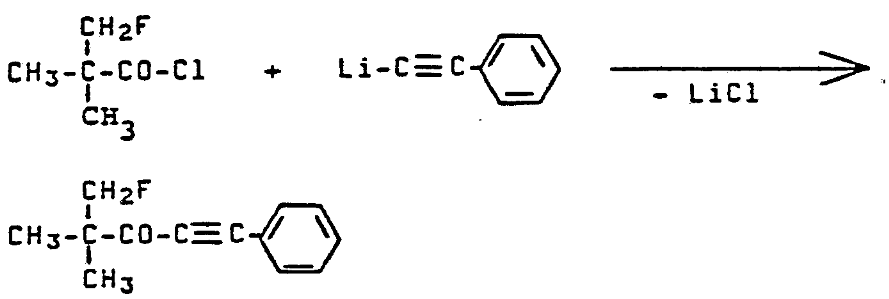


Verwendet man beispielsweise α,α-Bisfluormethylpropionsäurechlorid und Phenylacetylen als Ausgangsstoffe, Kupfer-(I)-bromid als Katalysator und Triethylamin als Base, so kann der Reaktionsablauf des erfindungsgemäßen Verfahrens durch das folgende Formelschema wiedergegeben werden :

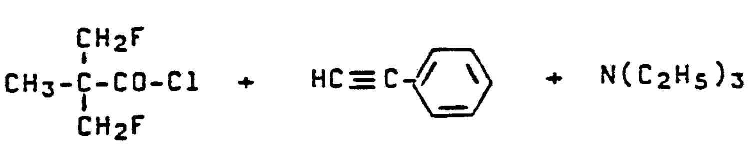

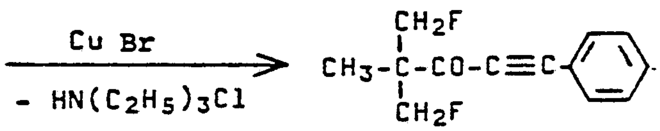


Die für die Durchführung des erfindungsgemäßen Verfahrens als Ausgangsstoffe zu verwendenden Acetylen-Derivate sind durch die Formel (II) allgemein definiert. In dieser Formel steht $R^2$ für die Bedeutungen, die bereits im Zusammenhang mit der Beschreibung der erfindungsgemäßen Stoffe der Formel (I) für diesen Substituenten genannt wurden. M steht vorzugsweise für Wasserstoff oder ein entsprechendes Äquivalent eines Alkali- oder Erdalkalimetalls, wie beispielsweise Lithium, Natrium oder Magnesium.

Die Acetylen-Derivate der Formel (II) sind allgemein bekannte Verbindungen der organischen Chemie, bzw. können sie in allgemein bekannter Art und Weise erhalten werden (vgl. hierzu z. B. Tetrahedron 37 (1981), 1653-1658 ; J. Chem. Research 1978, 106-107 ; 1979, 130 und 1981, 270-271 ; sowie Houben-Weyl, Methoden der organischen Chemie, Band V/2a, S. 351 ff.).

Die außerdem für das erfindungsgemäße Verfahren als Ausgangsstoffe zu verwendenden Säurederivate sind durch die Formel (III) allgemein definiert. In dieser Formel steht $R^1$ für die Bedeutungen, die bereits im Zusammenhang mit der Beschreibung der erfindungsgemäßen Stoffe der Formel (I) für diesen Substituenten genannt wurden. Y steht für eine elektronenanziehende Abgangsgruppierung, wie vorzugsweise Halogen, p-Methylphenylsulfonyloxy oder die Gruppierungen $—O—SO_2—OR$ und $—NR_3$, wobei R für einen gegebenenfalls substituierten Kohlenwasserstoffrest steht.

Die Säurederivate der Formel (III) sind allgemein bekannte Verbindungen der organischen Chemie, bzw. können sie in allgemein bekannter Art und Weise erhalten werden (vgl. hierzu z. B. US-A 34 14 612, DE-A 31 28 445, EP-A 00 49 416).

Als Verdünnungsmittel kommen für das erfindungsgemäße Verfahren inerte organische Lösungsmittel in Frage. Hierzu gehören vorzugsweise aromatische Kohlenwasserstoffe, wie Toluol, Xylol oder Chlorbenzol ; Nitrile wie Acetonitril ; Ether, wie Diisobutylether oder Dioxan ; sowie Pyridin.

Die Reaktionstemperaturen können bei der Durchführung des erfindungsgemäßen Verfahrens in einem größeren Bereich variiert werden. Im allgemeinen arbeitet man im Bereich zwischen 0 °C und 150 °C, vorzugsweise zwischen 20 °C und 120 °C.

Als Basen kommen für das erfindungsgemäße Verfahren vorzugsweise tertiäre Amine in Frage. Hierbei seien insbesondere genannt : Triethylamin, N,N-Dimethylcyclohexylamin und N,N-Dimethylbenzylamin.

Als katalytisch wirksame Substanzen kommen bei der Durchführung des erfindungsgemäßen Verfahrens vorzugsweise Kupfer-(I)-chlorid und Kupfer-(I)-bromid in Betracht.

Bei der Durchführung des erfindungsgemäßen Verfahrens setzt man die Verbindungen der Formeln (II) und (III) vorzugsweise in äquimolaren Mengen um. In vielen Fällen empfiehlt es sich, eine Base als Säurebindemittel und ein Kupfer-(I)-salz als katalytisch wirkende Substanz hinzuzufügen. Die Aufarbeitung und die Isolierung der Verbindungen der Formel (I) erfolgen nach üblichen Methoden.

Wie schon erwähnt, stellen die neuen substituierten Acetylenketone der Formel (I) interessante Zwischenprodukte zur Synthese von Wirkstoffen mit fungiziden und pflanzenwuchsregulierenden Eigenschaften dar. So lassen sich Azolyl-Ketone und -carbinole der Formel (IV)

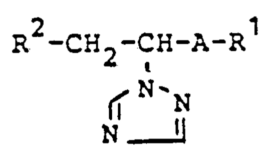 (IV)

in welcher

$R^1$ und $R^2$ die oben angegebenen Bedeutungen haben und

A für die Keto- oder die CH(OH)-Gruppe steht,

erhalten, indem man substituierte Acetylenketone der Formel (I)

$$R^2—C≡C—CO—R^1 \qquad (I)$$

in welcher $R^1$ und $R^2$ die oben angegebenen Bedeutungen haben,

selektiv (ohne Angriff der CO-Gruppe) mit Wasserstoff in Gegenwart eines Hydrierkatalysators und vorzugsweise in Gegenwart eines Verdünnungsmittels umsetzt ; anschließend die so erhaltenen Ketone der Formel (V)

$$R^2—CH_2—CH_2—CO—R^1 \qquad (V)$$

in welcher $R^1$ und $R^2$ die oben angegebenen Bedeutungen haben, mit Chlor oder Brom in Gegenwart eines inerten organischen Lösungsmittels, wie beispielsweise Ether, chlorierten oder nicht chlorierten Kohlenwasserstoffen, bei Raumtemperatur umsetzt, oder mit üblichen Chlorierungsmitteln, wie beispielsweise Sulfurylchlorid, bei 20 °C bis 60 °C umsetzt ; danach die so erhaltenen Halogenketone der Formel (VI)

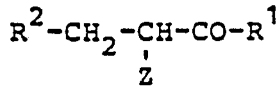

(VI)

in welcher

$R^1$ und $R^2$ die oben angegebenen Bedeutungen haben und

Z für Chlor oder Brom steht,

mit 1,2,4-Triazol in Gegenwart eines inerten organischen Lösungsmittels, wie beispielsweise Acetonitril, und in Gegenwart eines Säurebindemittels, wie beispielsweise Kaliumcarbonat oder in Gegenwart eines Überschusses an 1,2,4-Triazol bei Temperaturen zwischen 60 °C und 120 °C umsetzt ; und gegebenenfalls anschließend die so erhaltenen Azolyl-Ketone der Formel (IVa),

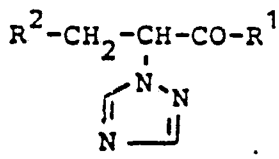

(IVa)

in welcher $R^1$ und $R^2$ die oben angegebenen Bedeutungen haben, durch Umsetzung mit komplexen Hydriden, wie Natriumborhydrid oder Lithiumalanat, in Gegenwart eine polaren organischen Lösungsmittels, wie z. B. eines Alkoholes, bei Temperaturen zwischen 0 °C bis 30 °C reduziert ; oder durch Umsetzung mit Aluminiumisopropylat in Gegenwart eines Verdünnungsmittels, wie z. B. Isopropanol, bei Temperaturen zwischen 20 °C bis 120 °C reduziert.

Die Hydrierungen in der ersten Stufe des oben beschriebenen Verfahrens werden in flüssiger Phase, vorzugsweise in Anwesenheit von Verdünnungsmitteln, unter Verwendung eines suspendierten, pulverförmigen Hydrierungskatalysators, durchgeführt. Die Durchführung der Hydrierungen kann diskontinuierlich (chargenweise) oder kontinuierlich als Sumpf- oder Rieselphasenhydrierung in bekannten Hydrierreaktoren, wie Autoklaven, Autoklavenkaskaden, Rohrreaktoren oder Umlaufreaktoren erfolgen. Die bevorzugte Arbeitsweise ist die diskontinuierliche Sumpfphasenhydrierung im Autoklaven bei erhöhtem Druck.

Als Verdünnungsmittel kommen für die Hydrierungen inerte organische Lösungsmittel in Frage. Hierzu gehören vorzugsweise Alkohole, wie Methanol, Ethanol, Isopropanol oder Ethylenglykol ; Ether, wie Diethylether, Diisopropylether, Ethylenglykolmonomethylether, Ethylenglykoldimethylether, Dioxan oder Tetrahydrofuran ; gesättigte Kohlenwasserstoffe, wie n-Heptan oder Cyclohexan ; sowie Ester, wie Essigsäureethylester.

Für die Hydrierungen geeignete Hydrierkatalysatoren sind beispielsweise solche, die aus Metallen und/oder Verbindungen von Elementen der achten Nebengruppe des periodischen Systems der Elemente nach Mendelejew bestehen oder diese enthalten. Dabei sind die Metalle Ruthenium, Rhodium, Palladium, Platin, Kobalt und Nickel und deren Verbindungen bevorzugt. Bei den Metallverbindungen kann es sich beispielsweise um Oxide, Hydroxide und/oder Oxihydrate handeln. Zusätzlich können die Metalle Kupfer, Vanadium, Molybdän, Chrom und/oder Mangan, sowie Verbindungen dieser Metalle zugegen sein.

Die Hydrierkatalysatoren können ausschließlich oder überwiegend aus Wasserstoff-übertragenden Substanzen bestehen ; diese können aber auch auf Trägermaterialien aufgebracht werden.

Als Trägermaterialien für die Wasserstoff-übertragenden Substanzen kommen beispielsweise in Frage : anorganische Materialien, wie Kieselgur, Kieselsäure, Aluminiumoxide, Alkali- und Erdalkalisilikate, Aluminiumsilikate, Montmorillonit, Zeolithe, Spinelle, Dolomit, Kaolin, Magnesiumsilikate, Zirkonoxid, Zinkoxid, Calciumcarbonat, Siliciumcarbid, Aluminiumphosphat, Borphosphat, Asbest, Aktivkohle oder Bariumsulfat, aber auch organische Materialien, beispielsweise natürlich vorkommende oder synthetische Verbindungen mit hohen Molekulargewichten, wie Seide, Polyamide, Polystyrole, Zellstoff oder Polyurethane. Bevorzugt sind anorganische Trägermaterialien in Pulverform.

Derartige Trägerkatalysatoren können im allgemeinen 0,5 bis 50 Gew.-%, vorzugsweise 1 bis 10 Gew.-% der Wasserstoff-übertragenden Substanzen, bezogen auf die Gesamtmasse des Trägerkatalysators, enthalten. Die Wasserstoff-übertragende Substanz kann dabei homogen im Trägermaterial verteilt sein, bevorzugt sind jedoch Katalysatoren, in deren äußerer Schicht oder auf deren Oberfläche die Wasserstoff-übertragende Substanz abgelagert ist. Die Herstellung und die Formgebung von Katalysatoren, die im erfindungsgemäßen Verfahren Verwendung finden können, kann in bekannter Weise erfolgen (vgl. beispielsweise Houben-Weyl, Methoden der organischen Chemie, Band IV, 1c, Teil I, S. 16-26, Georg Thieme Verlag, Stuttgart, 1980).

Bevorzugte Katalysatoren sind Ruthenium auf Kohle, Ruthenium auf Aluminiumoxid, Rhodium auf Kohle, Rhodium auf Aluminiumoxid, Palladium auf Calciumcarbonat, Palladium auf Bariumsulfat, Palladium auf Kieselsäure, Platin auf Kohle und Platin auf Aluminiumoxid, Nickel auf Kieselgur, Nickel auf Aluminiumoxid sowie Nickel und Palladium auf Aluminiumoxid.

Bevorzugte Hydrierkatalysatoren, die ausschließlich oder überwiegend aus Wasserstoff-übertragender Substanz bestehen, sind beispielsweise oxidische Katalysatoren, wie Palladiumoxid, Platinoxid, Rutheniumoxid und/oder Rhodiumoxid/Platinoxid nach Nishimura, ferner durch Reduktion von entsprechenden Metallsalzen oder Metallsalzgemischen mit Alkalihydriden, Alkalicarbonaten, Metallalkylen, Hydrazin, Formaldehyd, Wasserstoff oder elektropositiveren Metallen herstellbare Schwarzkatalysatoren, wie Palladium/Schwarz, Platin/Schwarz und Rhodium/Schwarz ; sowie Skelettkatalysatoren vom Raney-Typ, wie Raney-Nickel, Raney-Kobalt, Raney-Nickel-Kobalt, Raney-Nickel-Eisen, Raney-Nickel-Kupfer, Raney-Nickel-Eisen-Chrom, Raney-Nickel-Palladium und Raney-Nickel-Eisen-Vanadium.

Die Auswahl eines oder mehrerer der genannten Hydrierkatalysatoren richtet sich zweckmäßigerweise nach der Konstitution der zu hydrierenden Acetylenketone der Formel (I) und/oder den gewünschten Wirkstoffen der Formel (IV).

Die Hydrierkatalysatoren werden in einer solchen Menge eingesetzt, daß 0,05 bis 2,5, vorzugsweise 0,1 bis 1 Gew.-% Wasserstoff-übertragende Substanz bezogen auf das Gesamtgewicht des Reaktionsgemisches vorliegen.

Bei der Durchführung der Hydrierung können auch Gemische aus zwei oder mehreren der genannten Hydrierkatalysatoren verwendet werden.

Die Reaktionstemperaturen können bei der Hydrierung in einem größeren Bereich variiert werden. Im allgemeinen arbeitet man im Bereich zwischen 0 °C und 150 °C, vorzugsweise zwischen 20 °C und 120 °C.

Die Hydrierung wird vorzugsweise bei erhöhtem Druck durchgeführt. Im allgemeinen arbeitet man zwischen 1 und 150 bar, vorzugsweise bei 20 bis 120 bar.

Die Azolyl-Ketone und -carbinole der Formel (IV) weisen starke fungizide und pflanzenwuchsregulierende Eigenschaften auf (vgl. EP-A 00 31 911, EP-A 00 32 200, EP-A 00 55 833, EP-A 00 54 865 und DE-A 32 24 129).

Die Herstellung und die Verwendung der erfindungsgemäßen Substanzen geht aus den folgenden Beispielen hervor.

## Herstellungsbeispiele

### Beispiel 1

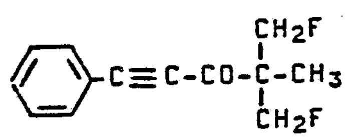

In 30 ml Pyridin legte man unter Stickstoff 10,1 g (0,1 Mol) Triethylamin und 1,43 g (0,01 Mol) Kupfer-(I)-bromid vor. Man fügte 10,2 g (0,1 Mol) Phenyl-acetylen zu und ließ 30 Minuten nachrühren. Danach versetzte man die Reaktionsmischung tropfenweise mit 15,6 g (0,1 Mol) α,α-Bisfluormethyl-propionsäurechlorid und hielt die Temperatur dabei auf 60 °C. Man ließ 15 Stunden bei dieser Temperatur rühren, kühlte dann ab, wusch mit Wasser, trocknete über Natriumsulfat und engte im Vakuum ein. Der Rückstand wurde durch Destillation gereinigt.

Man erhielt 17,3 g (78 % der Theorie) 2,2-Bisfluormethyl-5-phenyl-4-pentin-3-on vom Siedepunkt 103 °C bis 105 °C/0,2 mbar.

### Beispiel 2

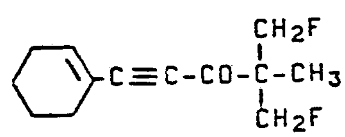

In 30 ml Pyridin legte man unter Stickstoff 10,1 g (0,1 Mol) Triethylamin und 1,43 g (0,01 Mol) Kupfer-(I)-bromid vor. Man fügte 10,6 g (0,1 Mol) Cyclohexen-1-yl-acetylen zu und ließ 20 Minuten nachrühren. Danach versetzte man die Reaktionsmischung tropfenweise mit 15,6 g (0,1 Mol) α,α-Bisfluormethylpropionsäurechlorid und hielt die Temperatur auf 70 °C. Man ließ 15 Stunden bei dieser Temperatur rühren, kühlte dann ab, wusch mit Wasser, trocknete über Natriumsulfat und engte im Vakuum ein. Der Rückstand wurde durch Destillation gereinigt.

Man erhielt 18,1 g (80 % der Theorie) 2,2-Bisfluormethyl-5-cyclohexen-1-yl-4-pentin-3-on vom Siedepunkt 106 °C bis 109 °C/0,3 mbar.

In analoger Weise und gemäß den angegebenen Verfahrensbedingungen wurden die folgenden Acetylenketone der Formel (I) erhalten :

Tabelle 1

$$R^2—C\equiv C—CO—R^1 \qquad (I)$$

| Beisp. Nr. | $R^1$ | $R^2$ | Fp.(°C) bzw. Kp.(°C)/mbar bzw. $n_D^{20}$ |
|---|---|---|---|
| 3 | $(ClCH_2)_2(CH_3)C$- | 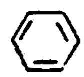 | 1,5465 |
| 4 | $(ClCH_2)_2(CH_3)C$- | 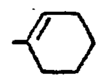 | 44 |
| 5 | Cl-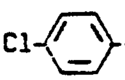-O-$CH_2$-C$(CH_3)_2$ | 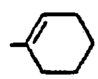 | 60 |
| 6 | $C_2H_5$-C$(CH_3)_2$ | 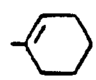 | 1,5202 |
| 7 | $FCH_2$-C$(CH_3)_2$ | 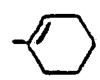 | 99 - 103 |
| 8 | $(FCH_2)_2(CH_3)C$- | $C_4H_9$ | 70-73/0,1 |

Herstellung von (1,2,4-Triazol-1-yl)-Derivaten der Formel (IV)

Beispiel 9

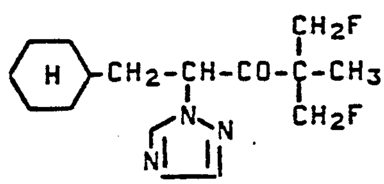

(IV-1)

1. Stufe

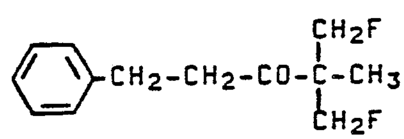

(V-1)

Ein 0,3 l Rührautoklav aus Edelstahl, der mit Hilfe eines regelbaren Thermostaten temperiert werden kann, wurde mit 4,4 g (0,2 Mol) 2,2-Bisfluormethyl-5-phenyl-4-petin-3-on (Beispiel 1), 170 ml Methanol und 5 g Raney-Nickel beschickt.

Nach Verschließen des Autoklaven und Verdrängen der Luft mit Stickstoff wurde das eingesetzte Gemisch mit Wasserstoff bis zu einem Druck von 30 bar beaufschlagt und unter Rühren auf 30 °C erhitzt. Sobald diese Temperatur erreicht war, wurde der Wasserstoffdruck auf 50 bar erhöht und nach Maßgabe des Wasserstoff-Verbrauches während der gesamten Reaktionszeit aufrechterhalten.

Nachdem die Wasserstoffaufnahme nach etwa 2 Stunden beendet war, wurde zur Vervollständigung der Umsetzung noch eine Stunde unter den vorangenannten Hydrierbedingungen weiter gerührt, anschließend auf Raumtemperatur abgekühlt und auf Normaldruck entspannt.

Die durch Filtration vom Katalysator abgetrennte Produktlösung wurde im Rotationsverdampfer vom

Methanol befreit.

Man erhielt 44,5 g (98,5 % der Theorie) 2,2-Bisfluormethyl-5-phenyl-3-pentanon als Öl mit einem Gehalt von 96,5 % (gaschromatographisch bestimmt).

2. Stufe

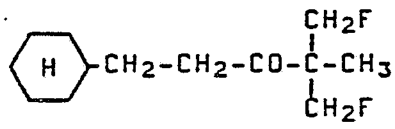

(V-2)

Ein 120 l Rührautoklav aus Edelstahl, der mit Hilfe eines regelbaren Thermostaten temperiert werden kann, wurde mit einer Lösung von 29,7 kg (131,4 Mol) 2,2-Bisfluormethyl-5-phenyl-3-pentanon in 60 Litern Methanol und 0,72 kg eines 5 % Ruthenium auf Aktivkohle enthaltenden Katalysators beschickt.

Nach Verschließen des Autoklaven und Verdrängen der Luft mit Stickstoff wurde das eingesetzte Gemisch mit Wasserstoff bis zu einem Druck von 50 bar beaufschlagt und unter Rühren auf 90 °C erhitzt. Sobald diese Temperatur erreicht war, wurde der Wasserstoffdruck auf 100 bar erhöht und nach Maßgabe des Wasserstoff-Verbrauches während der gesamten Reaktionszeit aufrechterhalten.

Nachdem die Wasserstoffaufnahme nach etwa 6 Stunden beendet war, wurde zur Vervollständigung der Umsetzung noch eine Stunde unter den vorangenannten Hydrierbedingungen weiter gerührt, anschließend auf Raumtemperatur abgekühlt und auf Normaldruck entspannt.

Die durch Filtration vom Katalysator abgetrennte Produktlösung wurde im Rotationsverdampfer vom Methanol befreit.

Man erhielt 30,3 kg (99,4 % der Theorie) 2,2-Bisfluormethyl-5-cyclohexyl-3-pentanon als Öl mit einem Gehalt von 96 % (gaschromatographisch bestimmt).

3. Stufe

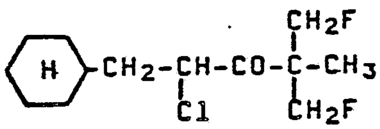

(VI-1)

232 g (1 Mol) 2,2-Bisfluormethyl-5-cyclohexyl-3-pentanon wurden auf 80 °C erhitzt und innerhalb einer Stunde tropfenweise mit 161,9 g (1,2 Mol) Sulfurylchlorid versetzt. Man ließ 5 Stunden bei 80 °C nachrühren und destillierte anschließend überschüssiges Sulfurylchlorid im Vakuum ab. Nach dem Abkühlen auf 20 °C wurde mit 500 ml Methylisobutylketon versetzt. Die organische Lösung wurde mit Wasser neutral gewaschen, über Magnesiumsulfat getrocknet und im Vakuum eingeengt. Der Rückstand wurde destilliert.

Man erhielt 252 g (90 % der Theorie) 2,2-Bisfluormethyl-4-chlor-5-cyclohexyl-3-pentanon vom Siedepunkt 118 °C bis 120 °C/2,5 mbar.

4. Stufe

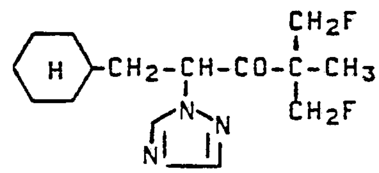

(IV-1)

Ein Gemisch aus 266,7 g (1 Mol) 2,2-Bisfluormethyl-4-chlor-5-cyclohexyl-3-pentanon, 69,1 g (1 Mol) 1,2,4-Triazol und 165,8 g (1,2 Mol) Kaliumcarbonat in 1 000 ml Methylisobutylketon wurde 6 Stunden unter Rückfluß erhitzt. Nach dem Abkühlen wurde mit verdünnter Salzsäure und mit Wasser neutral gewaschen. Die organische Phase wurde über Magnesiumsulfat getrocknet und im Vakuum eingeengt.

Man erhielt 329 g (88 % der Theorie) 2,2-Bisfluormethyl-5-cyclohexyl-4-(1,2,4-triazol-1-yl)-3-pentanon vom Brechungsindex $n_D^{20}$ : 1.4933.

Beispiel 10

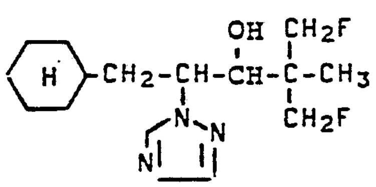

(IV-2)

1. Stufe

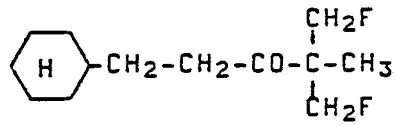

(V-1)

Ein 0,3 l Rührautoklav aus Edelstahl, der mit Hilfe eines regelbaren Thermostaten temperiert werden kann, wurde mit 24 g (0,106 Mol) 2,2-Bisfluormethyl-5-cyclohexen-1-yl-4-pentin-3-on (Beispiel 2), 120 ml Methanol und 5 g Raney-Nickel beschickt.

Nach Verschließen des Autoklaven und Verdrängen der Luft mit Stickstoff wurde das eingesetzte Gemisch mit Wasserstoff bis zu einem Druck von 50 bar beaufschlagt und unter Rühren auf 50 °C erhitzt. Sobald diese Temperatur erreicht war, wurde der Wasserstoffdruck auf 70 bar erhöht und nach Maßgabe des Wasserstoff-Verbrauches während der gesamten Reaktionszeit aufrechterhalten.

Nachdem die Wasserstoffaufnahme beendet war, wurde zur Vervollständigung der Umsetzung noch eine Stunde unter den vorangenannten Hydrierbedingungen weiter gerührt, anschließend auf Raumtemperatur abgekühlt und auf Normaldruck entspannt.

Die durch Filtration vom Katalysator abgetrennte Produktlösung wurde am Rotationsverdampfer vom Methanol befreit.

Man erhielt 23,5 g (95,5 % der Theorie) 2,2-Bisfluormethyl-5-cyclohexyl-3-pentanon als Öl mit einem Gehalt von 88,5 % (gaschromatographisch bestimmt).

2. Stufe

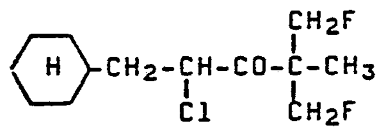

(VI-1)

Die Durchführung erfolgte entsprechend der 3. Stufe des Beispiels 11.

3. Stufe

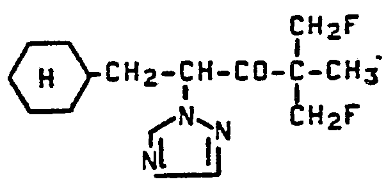

(IV-1)

Die Durchführung erfolgte entsprechend der 4. Stufe des Beispiels 11.

4. Stufe

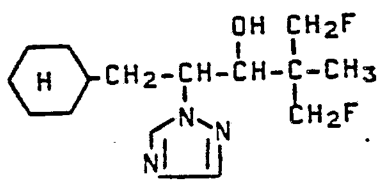

(IV-2)

299 g (1 Mol) 2,2-Bisfluormethyl-5-cyclohexyl-4-(1,2,4-triazol-1-yl)-3-pentanon wurden in 300 ml Methanol gelöst und bei 0 °C bis 5 °C tropfenweise mit einer Lösung von 13,2 g (0,35 Mol) Natriumborhydrid in 150 ml 0,1 normaler wäßriger Natronlauge versetzt. Nach einer Reaktionszeit von 2 Stunden wurde die Reaktionslösung mit verdünnter Salzsäure auf einen pH-Wert von 4 bis 5 eingestellt. Nach Zugabe von 500 ml Wasser kristallisierte das Endprodukt aus.

Nach Trocknung im Vakuum erhielt man 286 g (95 % der Theorie) 2,2-Bisfluormethyl-5-cyclohexyl-4-(1,2,4-triazol-1-yl)-3-pentanol vom Schmelzpunkt 103 °C bis 105 °C.

Vergleichsbeispiel

Herstellung des (1,2,4-Triazol-1-yl)-Derivates der Formel

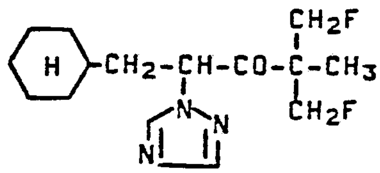

(IV-1)

nach dem bisher bekannten Verfahren.

1. Stufe

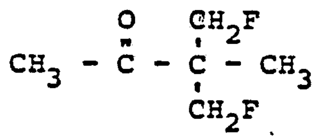

In einem Dreihalskolben mit Rührer, Tropftrichter und Liebigkühler mit gekühlter Vorlage wurden 400 ml Tetraethylenglykol und 46,4 g Kaliumfluorid (0,8 Mol) vorgelegt und auf 170 °C aufgeheizt. Man legte an den Vorstoß des Liebigkühlers ein Wasserstrahlvakuum (Druck ca. 20 bis 30 mbar) an. Dann wurden innerhalb von 45 Minuten 57,6 g (0,2 Mol) 2-Acetyl-2-methyl-propan-1,3-diol-bismethansulfat, gelöst in 100 ml Tetraethylenglykol, zugetropft. Das entstandene 3,3-Bisfluormethyl-butan-2-on wurde während der Reaktion in die gekühlte Vorlage abdestilliert. Nach dem Zutropfen wurde noch 1 Stunde bei 175 °C weiter destilliert. Das aufgefangene Destillat wurde anschließend redestilliert. Man erhielt 14 g (51,5 % der Theorie) 3,3-Bisfluormethylbutan-2-on vom Kp. 43 bis 46 °C/12 mbar.

2. Stufe

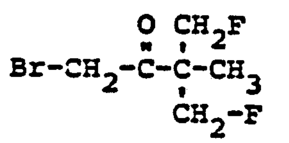

136 g (1 Mol) 3,3-Bisfluormethylbutan-2-on in 700 ml Methylenchlorid wurden bei Raumtemperatur tropfenweise so mit 51 ml (1 Mol) Brom versetzt, daß laufend Entfärbung eintrat. Anschließend wurde das Lösungsmittel im Wasserstrahlvakuum abdestilliert. Man erhielt nahezu quantitativ rohes 3,3-Bisfluormethyl-1-brom-butan-2-on als Öl, das direkt weiter umgesetzt werden konnte.

3. Stufe

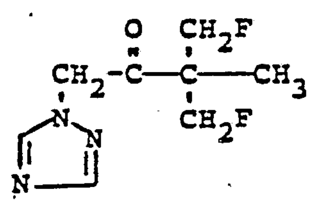

215 g (1 Mol) rohes 3,3-Bisfluormethyl-1-brom-butan-2-on wurden bei 30 bis 35 °C zu 84 g (1,2 Mol) 1,2,4-Triazol und 165 g (1,2 Mol) gemahlenem Kaliumcarbonat in 1 l Ethanol getropft. Man ließ über Nacht bei 40 °C nachrühren, filtrierte danach vom Ungelösten ab und engte das Filtrat ein. Der ölige Rückstand wurde mit Methylenchlorid und Wasser extrahiert, über Natriumsulfat getrocknet und eingeengt. Der Rückstand wurde in Methylenchlorid aufgenommen und mit 140 ml etherischer Salzsäure versetzt. Das entstandene kristalline Produkt wurde abgesaugt, mit 1 l Methylenchlorid und 1 l gesättigter, wäßriger Natriumhydrogencarbonatlösung ausgerührt, mit 1 l Wasser gewaschen, über Natriumsulfat getrocknet und eingeengt. Man erhielt 73,8 g (36,4 % der Theorie) 3,3-Bisfluormethyl-1-(1,2,4-triazol-1-yl)-butan-2-on als Öl, das direkt weiter umgesetzt werden konnte.

4. Stufe

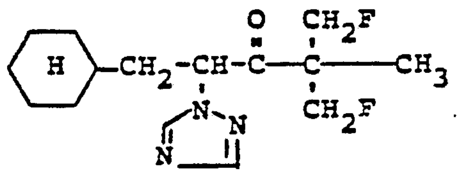

(IV-1)

Eine Lösung von 101,4 g (1,81 Mol) Kaliumhydroxid in 217,2 ml Wasser wurde bei Raumtemperatur unter Rühren in eine Lösung von 369,4 g (1,81 Mol) 2,2-Bis-fluormethyl-4-(1,2,4-triazol-1-yl)-butan-3-on in 2 Litern Diemthylsulfoxid gegeben. In dieses Gemisch wurden 320,5 g (1,81 Mol) Cyclohexylmethylbromid unter Rühren tropfenweise zugegeben, wobei die Temperatur des Reaktionsgemisches durch Kühlung zwischen 20 und 40 °C gehalten wurde. Das Reaktionsgemisch wurde noch 15 Stunden bei 60 °C gerührt und dann in 2 Liter Wasser gegossen. Man extrahierte das erhaltene Gemisch zweimal mit je 1 Liter Methylenchlorid, wusch die vereinigten organischen Phasen viermal mit je 2 Liter Wasser, trocknete über Natriumsulfat und zog das Lösungsmittel ab. Das verbleibende ölige Produkt wurde in Aceton aufgenommen, und 326 g Naphthalin-1,5-disulfonsäure wurden zu der Lösung hinzugefügt. Der sich dabei bildende Niederschlag wurde abgesaugt und in 2 Litern Methylenchlorid suspendiert. Diese Suspension wurde zweimal mit je 2 Litern gesättigter, wäßriger Natriumhydrogen-carbonat-Lösung geschüttelt. Dann wurde die organische Phase mit 2 Litern Wasser gewaschen und nach dem Trocknen über Natriumsulfat unter vermindertem Druck eingeengt. Man erhielt auf diese Weise 297,5 g (63 % der Theorie) an 2,2-Bis-fluormethyl-5-cyclohexyl-4-(1,2,4-triazol-1-yl)-pentan-3-on in Form eines Öles. $n_D^{20} = 1,4837$

**Patentansprüche**

1. Substituierte Acetylenketone der Formel

$$R^2—C \equiv C—CO—R^1 \qquad (I)$$

in welcher

$R^1$ für die Gruppierungen

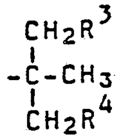 , 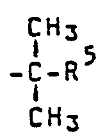 und 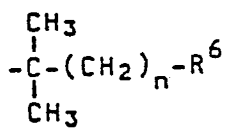 steht,

$R^2$ für geradkettiges oder verzweigtes Alkyl mit 1 bis 6 Kohlenstoffatomen, für jeweils gegebenenfalls einfach oder zweifach, gleich oder verschieden durch Methyl, Ethyl, Isopropyl und/oder tert.-Butyl substituiertes Cyclopropylmethyl, Cyclopentylmethyl, Cyclohexylmethyl, Cyclopentenyl, Cyclohexenyl oder Cycloheptenyl sowie für jeweils gegebenenfalls einfach oder zweifach gleich oder verschieden substituiertes Phenyl oder Phenylalkyl mit 1 bis 2 Kohlenstoffatomen im Alkylteil steht, wobei als Phenylsubstituenten jeweils Fluor, Chlor, Methyl, Ethyl, Isopropyl, tert.-Butyl, Methoxy, Methylthio, Trifluormethyl, Trifluormethoxy, Trifluormethylthio, Nitro, Cyano, Methoxycarbonyl, sowie jeweils gegebenenfalls durch Chlor oder Fluor substituiertes Phenyl und Phenoxy in Frage kommen,

$R^3$ für Fluor oder Chlor steht ;

$R^4$ für Wasserstoff, Fluor oder Chlor steht ;

$R^5$ für geradkettiges oder verzweigtes Alkyl mit 2 bis 6 Kohlenstoffatomen, Vinyl, Propargyl, die —CH=O-Gruppe, Methoximinomethyl, Dimethoxymethyl, den Dioxolan- oder 1,3-Dioxan-Rest steht ;

$R^6$ für Cyano oder gegebenenfalls einfach oder zweifach, gleich oder verschieden substituiertes Phenyl steht, wobei als Substituenten die bei $R^2$ bereits genannten Phenylsubstituenten in Frage, kommen, sowie für die Gruppierungen $—XR^7$ und $—CONR^8R^9$ steht ;

$R^7$ für geradkettiges oder verzweigtes Alkyl mit 1 bis 4 Kohlenstoffatomen, Halogenalkyl mit 1 bis 2 Kohlenstoffatomen und 1 bis 5 gleichen oder verschiedenen Halogenatomen, wie Fluor- und Chloratomen, sowie für jeweils gegebenenfalls einfach oder mehrfach, gleich oder verschieden substituiertes Phenyl und Benzyl steht, wobei jeweils als Substituenten die bei $R^2$ bereits genannten Phenylsubstituenten in Frage kommen ;

$R^8$ für Wasserstoff, Methyl, Ethyl, Isopropyl oder für gegebenenfalls einfach oder zweifach, gleich oder verschieden substituiertes Phenyl steht, wobei als Substituenten die bei $R^2$ bereits genannten Phenylsubstituenten in Frage kommen ;

$R^9$ für Wasserstoff, Methyl oder Isopropyl steht,

X für O, S, SO oder $SO_2$ steht und der Index

n für die Zahlen 0 oder 1 steht.

2. Substituiertes Acetylenketon der Formel

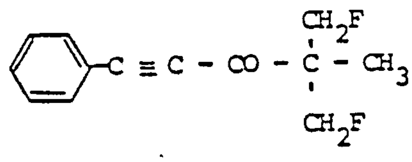

3. Substituiertes Acetylenketon der Formel

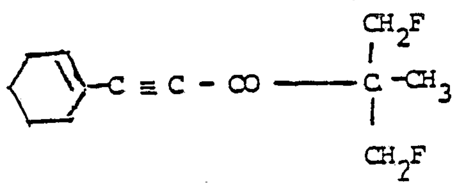

4. Substituiertes Acetylenketon der Formel

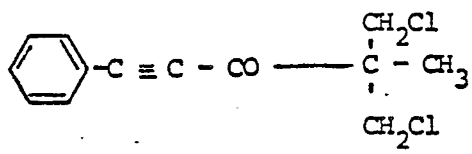

5. Substituiertes Acetylenketon der Formel

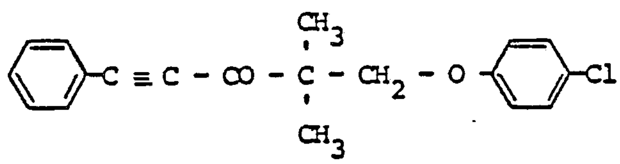

6. Substituiertes Acetylenketon der Formel

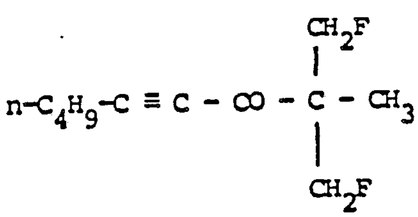

7. Verfahren zur Herstellung von substituierten Acetylenketonen der Formel

$$R^2—C{\equiv}C—CO—R^1 \qquad (I)$$

in welcher

$R^1$ für die Gruppierungen

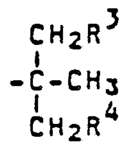 , 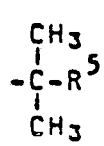 und 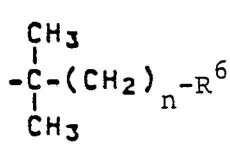 steht,

$R^2$ für geradkettiges oder verzweigtes Alkyl mit 1 bis 6 Kohlenstoffatomen, für jeweils gegebenenfalls einfach oder zweifach, gleich oder verschieden durch Methyl, Ethyl, Isopropyl und/oder tert.-Butyl substituiertes Cyclopropylmethyl, Cyclopentylmethyl, Cyclohexylmethyl, Cyclopentenyl, Cyclohexenyl oder Cycloheptenyl sowie für jeweils gegebenenfalls einfach oder zweifach gleich oder verschieden substituiertes Phenyl oder Phenylalkyl mit 1 bis 2 Kohlenstoffatomen im Alkylteil steht, wobei als Phenylsubstituenten jeweils Fluor, Chlor, Methyl, Ethyl, Isopropyl, tert.-Butyl, Methoxy, Methylthio, Trifluormethyl, Trifluormethoxy, Trifluormethylthio, Nitro, Cyano, Methoxycarbonyl, sowie jeweils gegebenenfalls durch Chlor oder Fluor substituiertes Phenyl und Phenoxy in Frage kommen,

$R^3$ für Fluor oder Chlor steht ;

$R^4$ für Wasserstoff, Fluor oder Chlor steht ;

$R^5$ für geradkettiges oder verzweigtes Alkyl mit 2 bis 6 Kohlenstoffatomen, Vinyl, Propargyl, die —CH=O-Gruppe, Methoximinomethyl, Dimethoxymethyl, den Dioxolan- oder 1,3-Dioxan-Rest steht ;

$R^6$ für Cyano oder gegebenenfalls einfach oder zweifach, gleich oder verschieden substituiertes Phenyl steht, wobei als Substituenten die bei $R^2$ bereits genannten Phenylsubstituenten in Frage kommen, sowie für die Gruppierungen $—XR^7$ und $—CONR^8R^9$ steht ;

$R^7$ für geradkettiges oder verzweigtes Alkyl mit 1 bis 4 Kohlenstoffatomen, Halogenalkyl mit 1 bis 2 Kohlenstoffatomen und 1 bis 5 gleichen oder verschiedenen Halogenatomen, wie Fluor- und Chloratomen, sowie für jeweils gegebenenfalls einfach oder mehrfach, gleich oder verschieden substituiertes Phenyl und Benzyl steht, wobei jeweils als Substituenten die bei $R^2$ bereits genannten Phenylsubstituenten in Frage kommen ;

$R^8$ für Wasserstoff, Methyl, Ethyl, Isopropyl oder für gegebenenfalls einfach oder zweifach, gleich oder verschieden substituiertes Phenyl steht, wobei als Substituenten die bei $R^2$ bereits genannten Phenylsubstituenten in Frage kommen ;

$R^9$ für Wasserstoff, Methyl oder Isopropyl steht ;

X für O, S, SO oder $SO_2$ steht und der Index

n für die Zahlen 0 oder 1 steht,

dadurch gekennzeichnet, daß man Acetylen-Derivate der Formel

$$R^2—C{\equiv}C—M \qquad (II)$$

in welcher

$R^2$ die oben angegebenen Bedeutungen hat und

M für ein Metall oder Wasserstoff steht,

mit Säurederivaten der Formel

$$Y—CO—R^1 \qquad (III)$$

in welcher

$R^1$ die oben angegebenen Bedeutungen hat und

Y für eine elektronenziehende Abgangsgruppierung steht,

gegebenenfalls in Gegenwart eines Verdünnungsmittels sowie gegebenenfalls in Gegenwart einer Base und von Kupfer-(I)-Ionen umsetzt.

8. Verfahren zur Herstellung von Azolyl-Ketonen und -carbinolen der Formel

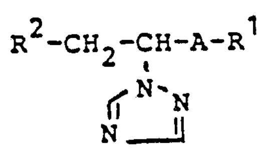

(IV)

in welcher

$R^1$ für die Gruppierungen

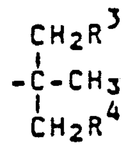 , 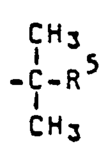 und 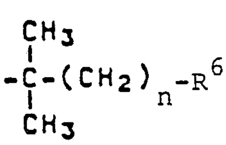 steht,

$R^2$ für geradkettiges oder verzweigtes Alkyl mit 1 bis 6 Kohlenstoffatomen, für jeweils gegebenenfalls einfach oder zweifach, gleich oder verschieden durch Methyl, Ethyl, Isopropyl und/oder tert.-Butyl substituiertes Cyclopropylmethyl, Cyclopentylmethyl, Cyclohexylmethyl, Cyclopentenyl, Cyclohexenyl oder Cycloheptenyl sowie für jeweils gegebenenfalls einfach oder zweifach gleich oder verschieden substituiertes Phenyl oder Phenylalkyl mit 1 bis 2 Kohlenstoffatomen im Alkylteil steht, wobei als Phenylsubstituenten jeweils Fluor, Chlor, Methyl, Ethyl, Isopropyl, tert.-Butyl, Methoxy, Methylthio, Trifluormethyl, Trifluormethoxy, Trifluormethylthio, Nitro, Cyano, Methoxycarbonyl, sowie jeweils gegebenenfalls durch Chlor oder Fluor substituiertes Phenyl und Phenoxy in Frage kommen,

$R^3$ für Fluor oder Chlor steht ;

$R^4$ für Wasserstoff, Fluor oder Chlor steht ;

$R^5$ für geradkettiges oder verzweigtes Alkyl mit 2 bis 6 Kohlenstoffatomen, Vinyl, Propargyl, die —CH=O-Gruppe, Methoximinomethyl, Dimethoxymethyl, den Dioxolan- oder 1,3-Dioxan-Rest steht ;

$R^6$ für Cyano oder gegebenenfalls einfach oder zweifach, gleich oder verschieden substituiertes Phenyl steht, wobei als Substituenten die bei $R^2$ bereits genannten Phenylsubstituenten in Frage

kommen, sowie für die Gruppierungen $—XR^7$ und $—CONR^8R^9$ steht ;

$R^7$ für geradkettiges oder verzweigtes Alkyl mit 1 bis 4 Kohlenstoffatomen, Halogenalkyl mit 1 bis 2 Kohlenstoffatomen und 1 bis 5 gleichen oder verschiedenen Halogenatomen, wie Fluor- und Chloratomen, sowie für jeweils gegebenenfalls einfach oder mehrfach, gleich oder verschieden substituiertes Phenyl und Benzyl steht, wobei jeweils als Substituenten die bei $R^2$ bereits genannten Phenylsubstituenten in Frage kommen ;

$R^8$ für Wasserstoff, Methyl, Ethyl, Isopropyl oder für gegebenenfalls einfach oder zweifach, gleich oder verschieden substituiertes Phenyl steht, wobei als Substituenten die bei $R^2$ bereits genannten Phenylsubstituenten in Frage kommen ;

$R^9$ für Wasserstoff, Methyl oder Isopropyl steht ;

X für O, S, SO oder $SO_2$ steht, der Index

n für die Zahlen 0 oder 1 steht, und

A für die Keto- oder die CH(OH)-Gruppe steht,

dadurch gekennzeichnet, daß man Acetylenketone der Formel

$$R^2—C{\equiv}C—CO—R^1 \qquad (I)$$

in welcher $R^1$ und $R^2$ die oben angegebenen Bedeutungen haben,
selektiv mit Wasserstoff in Gegenwart eines Hydrierkatalysators gegebenenfalls in Gegenwart eines Verdünnungsmittels umsetzt, dann die dabei anfallenden Ketone der Formel

$$R^2—CH_2—CH_2—CO—R^1 \qquad (V)$$

in welcher $R^1$ und $R^2$ die oben angegebenen Bedeutungen haben,
mit Chlor oder Brom in Gegenwart eines inerten organischen Lösungsmittels, oder mit Chlorierungsmitteln gegebenenfalls in Gegenwart eines Verdünnungsmittels umsetzt, danach die so erhaltenen Halogenketone der Formel

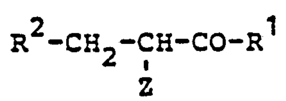

(VI)

in welcher

$R^1$ und $R^2$ die oben angegebenen Bedeutungen haben und

Z für Chlor oder Brom steht,

mit 1,2,4-Triazol in Gegenwart eines inerten organischen Lösungsmittels umsetzt, und gegebenenfalls anschließend die so erhaltenen Azolyl-Ketone der Formel

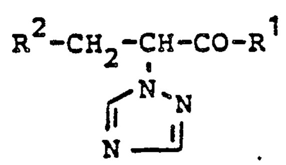

(IVa)

in welcher $R^1$ und $R^2$ die oben angegebenen Bedeutungen haben,
entweder mit komplexen Hydriden in Gegenwart eines polaren organischen Lösungsmittels, oder mit Aluminiumisopropylat in Gegenwart eines Verdünnungsmittels umsetzt.

**Claims**

1. Substituted acetylene-ketones of the formula

$$R^2—C{\equiv}C—CO—R^1 \qquad (I)$$

in which

$R^1$ represents

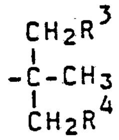 , 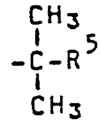 or 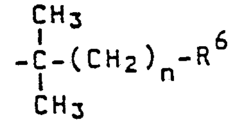

$R^2$ represents straight-chain or branched alkyl having 1 to 6 carbon atoms, or represents

cyclopropylmethyl, cyclopentylmethyl, cyclohexylmethyl, cyclopentenyl, cyclohexenyl or cycloheptenyl, in each case optionally mono- or disubstituted by identical or different methyl, ethyl, isopropyl and/or tert-butyl radicals, or represents phenyl or phenylalkyl having 1 to 2 carbon atoms in the alkyl part, in each case optionally mono or disubstituted, identically or differently, possible phenyl substituents in each case being fluorine, chlorine, methyl, ethyl, isopropyl, tert-butyl, methoxy, methylthio, trifluoromethyl, trifluoromethoxy, trifluoromethylthio, nitro, cyano and methoxycarbonyl and also phenyl and phenoxy which are in each case optionally substituted by chlorine or fluorine ;

$R^3$ represents fluorine or chlorine ;

$R^4$ represents hydrogen, fluorine or chlorine ;

$R^5$ represents straight-chain or branched alkyl having 2 to 6 carbon atoms, vinyl, propargyl, the —CH=O-group, methoximinomethyl, dimethoxymethyl, the dioxolane or the 1,3-dioxane radical ;

$R^6$ represents cyano, or phenyl which is optionally mono- or disubstituted, identically or differently, possible substituents being those phenyl substituents already mentioned for $R^2$, or represents the groupings $—XR^7$ or $—CONR^8R^9$ ;

$R^7$ represents straight-chain or branched alkyl having 1 to 4 carbon atoms, halogenoalkyl having 1 to 2 carbon atoms and 1 to 5 identical or different halogen atoms, such as fluorine and chlorine atoms, or represents phenyl or benzyl which are in each case optionally mono- or polysubstituted, identically or differently, possible substiuents in each case being those phenyl substitutents already mentioned for $R^2$ ;

$R^8$ represents hydrogen, methyl, ethyl or isopropyl, or phenyl which is optionally mono- or disubstituted, identically or differently, possible substituents being those phenyl substituents already mentioned for $R^2$ ;

$R^9$ represents hydrogen, methyl or isopropyl ;

X represents O, S, SO or $SO_2$ and the index n represents the numbers 0 or 1.

2. Substituted acetylene-ketone of the formula

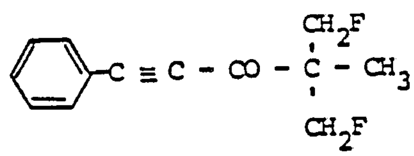

3. Substituted acetylene-ketone of the formula

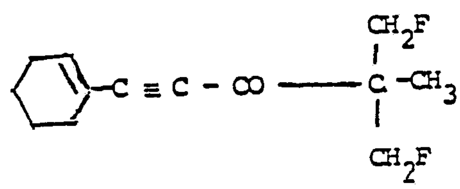

4. Substituted acetylene-ketone of the formula

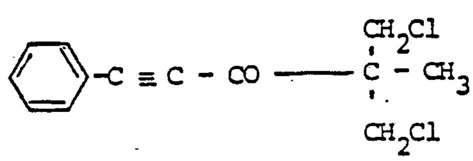

5. Substituted acetylene-ketone of the formula

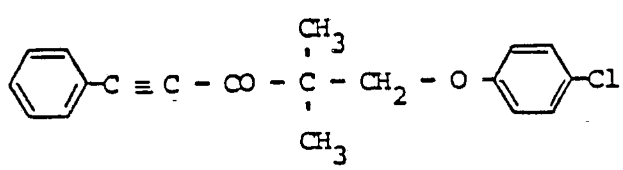

6. Substituted acetylene-ketone of the formula

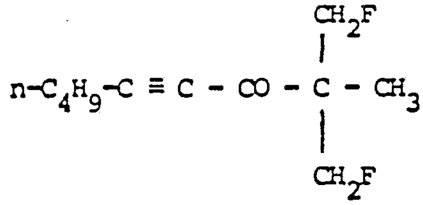

7. Process for the preparation of substituted acetylene-ketones of the formula

$$R^2—C≡C—CO—R^1 \quad (I)$$

in which

$R^1$ represents groupings

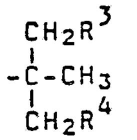 , 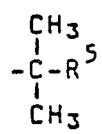 or 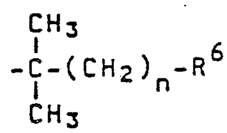

$R^2$ represents straight-chain or branched alkyl having 1 to 6 carbon atoms, or represents cyclopropylmethyl, cyclopentylmethyl, cyclohexylmethyl, cyclopentenyl, cyclohexenyl or cycloheptenyl, in each case optionally mono- or disubstituted by identical or different methyl, ethyl, isopropyl and/or tert-butyl radicals, or represents phenyl or phenylalkyl having 1 to 2 carbon atoms in the alkyl part, in each case optionally mono or disubstituted, identically or differently, possible phenyl substituents in each case being fluorine, chlorine, methyl, ethyl, isopropyl, tert-butyl, methoxy, methylthio, trifluoromethyl, trifluoromethoxy, trifluoromethylthio, nitro, cyano and methoxycarbonyl and also phenyl and phenoxy which are in each case optionally substituted by chlorine or fluorine ;

$R^3$ represents fluorine or chlorine ;

$R^4$ represents hydrogen, fluorine or chlorine ;

$R^5$ represents straight-chain or branched alkyl having 2 to 6 carbon atoms, vinyl, propargyl, the —CH=O-group, methoximinomethyl, dimethoxymethyl, the dioxolane or the 1,3-dioxane radical ;

$R^6$ represents cyano, or phenyl which is optionally mono- or disubstituted, identically or differently, possible substituents being those phenyl substituents already mentioned for $R^2$, or represents the groupings $—XR^7$ or $—CONR^8R^9$ ;

$R^7$ represents straight-chain or branched alkyl having 1 to 4 carbon atoms, halogenoalkyl having 1 to 2 carbon atoms and 1 to 5 identical or different halogen atoms, such as fluorine and chlorine atoms, or represents phenyl or benzyl which are in each case optionally mono- or polysubstituted, identically or differently, possible substituents in each case being those phenyl substituents already mentioned for $R^2$ ;

$R^8$ represents hydrogen, methyl, ethyl or isopropyl, or phenyl which is optionally mono- or disubstituted, identically or differently, possible substituents being those phenyl substituents already mentioned for $R^2$ ;

$R^9$ represents hydrogen, methyl or isopropyl ;

X represents O, S, SO or $SO_2$ and the index n represents the numbers 0 or 1,

characterized in that acetylene derivatives of the formula

$$R^2—C≡C—M \quad (II)$$

in which

$R^2$ has the abovementioned meanings and

M represents a metal or hydrogen,

are reacted with acid derivatives of the formula

$$Y—CO—R^1 \quad (III)$$

in which

$R^1$ has the abovementioned meanings and

Y represents an electron-withdrawing leaving grouping,

if appropriate in the presence of a diluent, and if appropriate in the presence of a base and of copper-(I) ions.

8. Process for the preparation of azolyl-ketones and -carbinols of the formula

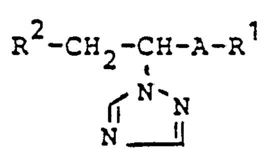 (IV)

in which

$R^1$ represents the groupings

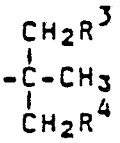 , 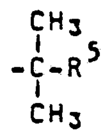 or 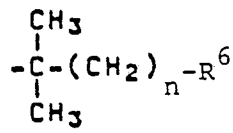

$R^2$ represents straight-chain or branched alkyl having 1 to 6 carbon atoms, or represents cyclopropylmethyl, cyclopentylmethyl, cyclohexylmethyl, cyclopentenyl, cyclohexenyl or cycloheptenyl, in each case optionally mono- or disubstituted by identical or different methyl, ethyl, isopropyl and/or tert-butyl radicals, or represents phenyl or phenylalkyl having 1 to 2 carbon atoms in the alkyl part, in each case optionally mono or disubstituted, identically or differently, possible phenyl substituents in each case being fluorine, chlorine, methyl, ethyl, isopropyl, tert-butyl, methoxy, methylthio, trifluoromethyl, trifluoromethoxy, trifluoromethylthio, nitro, cyano and methoxycarbonyl and also phenyl and phenoxy which are in each case optionally substituted by chlorine or fluorine ;

$R^3$ represents fluorine or chlorine ;

$R^4$ represents hydrogen, fluorine or chlorine ;

$R^5$ represents straight-chain or branched alkyl having 2 to 6 carbon atoms, vinyl, propargyl, the —CH=O-group, methoximinomethyl, dimethoxymethyl, the dioxolane or the 1,3-dioxane radical ;

$R^6$ represents cyano, or phenyl which is optionally mono- or disubstituted, identically or differently, possible substituents being those phenyl substituents already mentioned for $R^2$, or represents the groupings $—XR^7$ or $—CONR^8R^9$ ;

$R^7$ represents straight-chain or branched alkyl having 1 to 4 carbon atoms, halogenoalkyl having 1 to 2 carbon atoms and 1 to 5 identical or different halogen atoms, such as fluorine and chlorine atoms, or represents phenyl or benzyl which are in each case optionally mono- or polysubstituted, identically or differently, possible substituents in each case being those phenyl substituents already mentioned for $R^2$ ;

$R^8$ represents hydrogen, methyl, ethyl or isopropyl, or phenyl which is optionally mono- or disubstituted, identically or differently, possible substituents being those phenyl substituents already mentioned for $R^2$ ;

$R^9$ represents hydrogen, methyl or isopropyl ;

X represents O, S, SO or $SO_2$,

the index n represents the numbers 0 or 1 and

A represents the keto group or the CH(OH) group,

characterized in that acetylene-ketones of the formula

$$R^2—C≡C—CO—R^1 \qquad (I)$$

in which $R^1$ and $R^2$ have the abovementioned meanings,
are reacted selectively with hydrogen in the presence of a hydrogenation catalyst and if appropriate in the presence of a diluent, the ketones thereby obtained, of the formula

$$R^2—CH_2—CH_2—CO—R^1 \qquad (V)$$

in which $R^1$ and $R^2$ have the abovementioned meanings,
are then reacted with chlorine or bromine in the presence of an inert organic solvent, or with chlorinating agents, if appropriate in the presence of a diluent, the halogenoketones thus obtained, of the formula

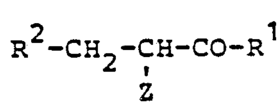

(VI)

in which

$R^1$ and $R^2$ have the abovementioned meanings and

Z represents chlorine or bromine,

are subsequently reacted with 1,2,4-triazole in the presence of an inert organic solvent, and, if appropriate, the azolyl-ketones thus obtained, of the formula

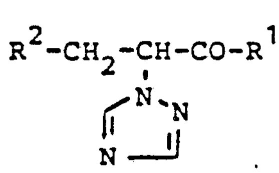

(IVa)

in which $R^1$ and $R^2$ have the abovementioned meanings,
are then reacted either with complex hydrides in the presence of a polar organic solvent or with aluminium isopropylate in the presence of a diluent.

**Revendications**

1. Acétylène-cétones substituées de formule

$$R^2—C\equiv C—CO—R^1 \qquad (I)$$

dans laquelle

$R^1$ représente les groupements

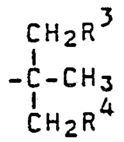 , 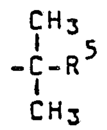 et 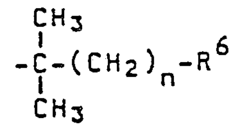

$R^2$ est un groupe alkyle à chaîne droite ou ramifié ayant 1 à 6 atomes de carbone, un groupe cyclopropylméthyle, cyclopentylméthyle, cyclohexylméthyle, cyclopenténylе, cyclohexényle ou cycloheptényle, chacun portant le cas échéant un ou deux substituants méthyle, éthyle, isopropyle et/ou tertiobutyle identiques ou différents, ainsi qu'un groupe phényle ou phénylalkyle avec 1 ou 2 atomes de carbone dans la partie alkyle, portant chacun éventuellement 1 ou 2 substituants identiques ou différents, et on considère alors comme substituants du groupe phényle, des substituants fluor, chlore, méthyle, éthyle, isopropyle, tertiobutyle, méthoxy, méthylthio, trifluorométhyle, trifluorométhoxy, trifluorométhylthio, nitro, cyano, méthoxycarbonyle, ainsi qu'un groupe phényle et un groupe phénoxy substitué chacun le cas échéant par du chlore ou du fluor ;

$R^3$ est du fluor ou du chlore ;

$R^4$ représente l'hydrogène, le fluor ou le chlore ;

$R^5$ est un groupe alkyle à chaîne droite ou ramifié de 2 à 6 atomes de carbone, un groupe vinyle, propargyle, le groupe —CH=O, un groupe méthoximinométhyle, diméthoxyméthyle, le reste dioxolanne ou 1,3-dioxanne ;

$R^6$ est un groupe cyano ou un groupe phényle portant le cas échéant un ou deux substituants identiques ou différents, et on considère alors comme substituants les substituants du groupe phényle déjà mentionnés pour $R^2$, ainsi que les groupements $—XR^7$ et $—CONR^8R^9$ ;

$R^7$ est un groupe alkyle à chaîne droite ou ramifié ayant 1 à 4 atomes de carbone, un groupe halogénalkyle ayant 1 ou 2 atomes de carbone et 1 à 5 atomes d'halogènes identiques ou différents, tels que des atomes de fluor et de chlore, ainsi que les groupes phényle et benzyle portant chacun, le cas échéant, un ou plusieurs substituants identiques ou différents, et on considère alors comme substituants les substituants du groupe phényle qui ont déjà été mentionnés pour $R^2$ ;

$R^8$ est l'hydrogène, un groupe méthyle, éthyle, isopropyle, ou un groupe phényle portant éventuellement un ou deux substituants identiques ou différents, et on considère alors comme substituants les substituants du groupe phényle déjà mentionnés pour $R^2$ ;

$R^9$ est l'hydrogène, un groupe méthyle ou isopropyle ;

X représente O, S, SO ou $SO_2$ et l'indice

n représente les nombres 0 ou 1.

2. Acétylène-cétone substituée de formule

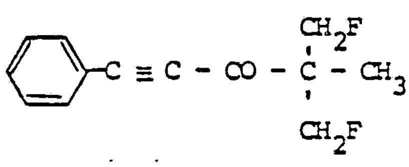

3. Acétylène-cétone substituée de formule

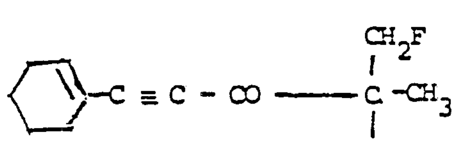

4. Acétylène-cétone substituée de formule

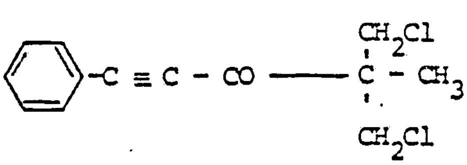

5. Acétylène-cétone substituée de formule

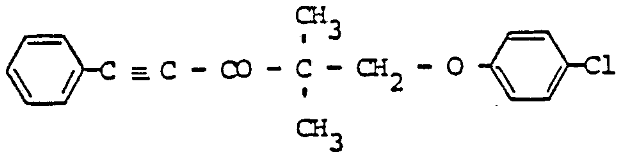

6. Acétylène-cétone substituée de formule

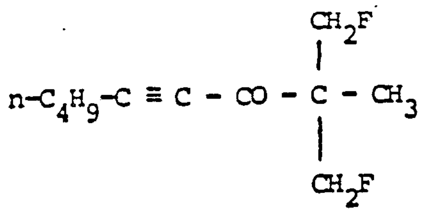

7. Procédé de production d'acétylène-cétones substituées de formule

$$R^2—C{\equiv}C—CO—R^1 \qquad (I)$$

dans laquelle

$R^1$ représente les groupements

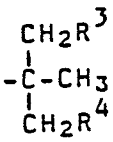 , 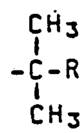 et 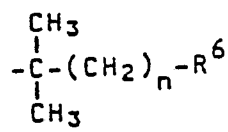

$R^2$ est un groupe alkyle à chaîne droite ou ramifié ayant 1 à 6 atomes de carbone, un groupe cyclopropylméthyle, cyclopentylméthyle, cyclohexylméthyle, cyclopentényle, cyclohexényle ou cycloheptényle, chacun portant le cas échéant un ou deux substituants méthyle, éthyle, isopropyle et/ou tertiobutyle identiques ou différents, ainsi qu'un groupe phényle ou phénylalkyle avec 1 ou 2 atomes de carbone dans la partie alkyle, portant chacun éventuellement 1 ou 2 substituants identiques ou différents, et on considère alors comme substituants du groupe phényle, des substituants fluor, chlore, méthyle, éthyle, isopropyle, tertiobutyle, méthoxy, méthylthio, trifluorométhyle, trifluorométhoxy, trifluorométhylthio, nitro, cyano, méthoxycarbonyle, ainsi qu'un groupe phényle et un groupe phénoxy substitué chacun le cas échéant, par du chlore ou du fluor ;

$R^3$ est du fluor ou du chlore ;

$R^4$ représente l'hydrogène, le fluor ou le chlore ;

$R^5$ est un groupe alkyle à chaîne droite ou ramifié de 2 à 6 atomes de carbone, un groupe vinyle, propargyle, le groupe —CH=O, un groupe méthoximinométhyle, diméthoxyméthyle, le reste dioxolanne ou 1,3-dioxanne ;

$R^6$ est un groupe cyano ou un groupe phényle portant le cas échéant un ou deux substituants identiques ou différents, et on considère alors comme substituants les substituants du groupe phényle déjà mentionnés pour $R^2$, ainsi que les groupements $—XR^7$ et $—CONR^8R^9$ ;

$R^7$ est un groupe alkyle à chaîne droite ou ramifié ayant 1 à 4 atomes de carbone, un groupe halogénalkyle ayant 1 ou 2 atomes de carbone et 1 à 5 atomes d'halogènes identiques ou différents, tels que des atomes de fluor et de chlore, ainsi que les groupes phényle et benzyle portant chacun, le cas échéant, un ou plusieurs substituants identiques ou différents, et on considère alors comme substituants les substituants du groupe phényle qui ont déjà été mentionnés pour $R^2$ ;

$R^8$ est l'hydrogène, un groupe méthyle, éthyle, isopropyle, ou un groupe phényle portant éventuellement un ou deux substituants identiques ou différents, et on considère alors comme substituants les substituants du groupe phényle déjà mentionnés pour $R^2$ ;

$R^9$ est l'hydrogène, un groupe méthyle ou isopropyle ;

X représente O, S, SO ou $SO_2$ et l'indice

n représente les nombres 0 ou 1,

caractérisé en ce qu'on fait réagir des dérivés d'acétylène de formule

$$R^2—C{\equiv}C—M \qquad (II)$$

dans laquelle

$R^2$ a les définitions indiquées ci-dessus et

M représente un métal ou l'hydrogène, avec des dérivés d'acides de formule

$$Y—CO—R^1 \qquad (III)$$

dans laquelle

$R^1$ a les définitions indiquées ci-dessus et

Y représente un groupement électro-attractif partant, éventuellement en présence d'un diluant ainsi que, le cas échéant, en présence d'une base et d'ions cuivre-(I).

8. Procédé de production d'azolyl-cétones et d'azolyl-carbinols de formule

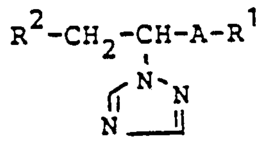

(IV)

dans laquelle

$R^1$ représente les groupements

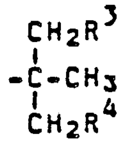 , 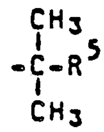 et 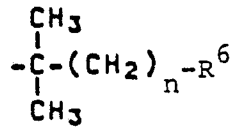

$R^2$ est un groupe alkyle à chaîne droite ou ramifié ayant 1 à 6 atomes de carbone, un groupe cyclopropylméthyle, cyclopentylméthyle, cyclohexylméthyle, cyclopenténylе, cyclohexényle ou cycloheptényle, chacun portant le cas échéant un ou deux substituants méthyle, éthyle, isopropyle et/ou tertiobutyle identiques ou différents, ainsi qu'un groupe phényle ou phénylalkyle avec 1 ou 2 atomes de carbone dans la partie alkyle, portant chacun éventuellement 1 ou 2 substituants identiques ou différents, et on considère alors comme substituants du groupe phényle, des substituants fluor, chlore, méthyle, éthyle, isopropyle, tertiobutyle, méthoxy, méthylthio, trifluorométhyle, trifluorométhoxy, trifluorométhylthio, nitro, cyano, méthoxycarbonyle, ainsi qu'un groupe phényle et un groupe phénoxy substitué chacun le cas échéant par du chlore ou du fluor ;

$R^3$ est du fluor ou du chlore ;

$R^4$ représente l'hydrogène, le fluor ou le chlore ;

$R^5$ est un groupe alkyle à chaîne droite ou ramifié de 2 à 6 atomes de carbone, un groupe vinyle, propargyle, le groupe $—CH=O$, un groupe méthoximinométhyle, diméthoxyméthyle, le reste dioxolanne ou 1,3-dioxanne ;

$R^6$ est un groupe cyano ou un groupe phényle portant le cas échéant un ou deux substituants identiques ou différents, et on considère alors comme substituants les substituants du groupe phényle déjà mentionnés pour $R^2$, ainsi que les groupements $—XR^7$ et $—CONR^8R^9$ ;

$R^7$ est un groupe alkyle à chaîne droite ou ramifié ayant 1 à 4 atomes de carbone, un groupe halogénalkyle ayant 1 ou 2 atomes de carbone et 1 à 5 atomes d'halogènes identiques ou différents, tels que des atomes de fluor ou de chlore, ainsi que les groupes phényle et benzyle portant chacun, le cas échéant, un ou plusieurs substituants identiques ou différents, et on considère alors comme substituants les substituants du groupe phényle qui ont déjà été mentionnés pour $R^2$ ;

$R^8$ est l'hydrogène, un groupe méthyle, éthyle, isopropyle, ou un groupe phényle portant éventuellement un ou deux substituants identiques ou différents, et on considère alors comme substituants les substituants du groupe phényle déjà mentionnés pour $R^2$ ;

$R^9$ est l'hydrogène, un groupe méthyle ou isopropyle ;

X représente O, S, SO ou $SO_2$ et l'indice

n représente les nombres 0 ou 1, et

A représente le groupe céto ou le groupe CH(OH),

caractérisé en ce qu'on fait réagir des acétylène-cétones de formule

$$R^2—C{\equiv}C—CO—R^1 \qquad (I)$$

dans laquelle $R^1$ et $R^2$ ont les définitions indiquées ci-dessus, sélectivement avec de l'hydrogène en présence d'un catalyseur d'hydrogénation, le cas échéant en présence d'un diluant, puis on fait réagir les cétones ainsi obtenues de formule

$$R^2—CH_2—CH_2—CO—R^1 \qquad (V)$$

dans laquelle $R^1$ et $R^2$ ont les définitions indiquées ci-dessus, avec le chlore ou le brome en présence d'un solvant organique inerte ou avec des agents de chloration, le cas échéant en présence d'un diluant, après quoi on fait réagir les halogénocétones ainsi obtenues de formule

$$R^2-CH_2-\underset{\substack{| \\ Z}}{CH}-CO-R^1 \qquad (VI)$$

dans laquelle

$R^1$ et $R^2$ ont les définitions indiquées ci-dessus et

Z représente le chlore ou le brome,

avec le 1,2,4-triazole en présence d'un solvant organique inerte, puis le cas échéant, on fait réagir les azolylcétones ainsi obtenues de formule

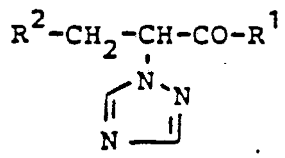

(IVa)

dans laquelle $R^1$ et $R^2$ ont les définitions indiquées ci-dessus, soit avec des hydrures complexes en présence d'un solvant organique polaire, soit avec l'isopropylate d'aluminium en présence d'un diluant.